# FASCICULE DE BREVET EUROPEEN

(11) **EP 0 836 609 B1**
(45) Date de publication et mention de la délivrance du brevet: **02.03.2005**
(21) Numéro de dépôt: 97920461.7
(22) Date de dépôt: 30.04.1997
(51) Int. Cl.: C07H 5/02, C07B 59/00

(54) **PROCEDE ET DISPOSITIF DE SYNTHESE DE COMPOSES MARQUES**
VERFAHREN UND VORRICHTUNG ZUR SYNTHESE MARKIERTER VERBINDUNGEN.
METHOD AND DEVICE FOR SYNTHESISING LABELLED COMPOUNDS

(30) Priorité: 02.05.1996 BE 9600388
(43) Date de publication de la demande: 22.04.1998
(60) Demande divisionnaire: 04030916.3
(73) Titulaire: GE Medical Systems Benelux SA, 1831 Diegem (Machelen) (BE); UNIVERSITE LIBRE DE BRUXELLES, 1050 Bruxelles (BE); Université de Liège, 4000 Liège (BE)
(72) Inventeur: DAMHAUT, Philippe, E., B-4032 Chenee (BE); MONCLUS, Michel, B-7050 Jurbise (BE); VAN NAMEN, John, J., B-9500 Geraardsbergen (BE); MULLENEERS, Eric, B-1480 Tubize (BE); MORELLE, Jean-Luc, E., B-1050 Bruxelles (BE); LEMAIRE, Christian, F., B-4432 Alleur (BE); LUXEN, André, B-4560 Ocquier (BE); LAURICELLA, Benjamin, P., B-4430 Ans (BE)
(74) Mandataire: Van Malderen, Joelle
(86) Numéro de dépôt international: PCT/BE1997/000056
(87) Numéro de publication internationale: WO 1997/042203

(56) Documents cités:
- EP-A- 0 588 480
- EP-A- 0 798 307
- WO-A-94/21653
- JP-A- 7 553 596
- JP-A- 9 263 593
- CARBOHYDRATE RESEARCH, vol. 175, no. 1, 1988, AMSTERDAM NL, pages 49-58, XP002038636 M. J. KING-MORRIS & P. B. BONDO: "Stable, isotopically substituted carbohydrates: an improved synthesis of (6-13C)aldohexoses."
- JOURNAL OF LABELLED COMPOUNDS AND RADIOPHARMACEUTICALS, vol. 38, no. 9, 10 Avril 1996, pages 809-824, XP002038637 N. MINAKAWA ET AL: "Syntheses of [2-2H]-5-ethynyl-1-(beta-D-ribofuranosyl)i midazole-4-carboxamide and 5-ethynyl-1-([5-3H]-beta-D-ribofuranosyl)i midazole-4-carboxamide (EICAR)"
- NUCL. MED. BIOL., vol. 17, no. 3, 1990, pages 273-279, XP000676335 S. A. TOORONGIAN ET AL: "Routine production of 2-deoxy-2-[18F]fluoro-D-glucose by direct nucleophilic exchange on a quaternary 4-aminopyridinium resin."
- PATENT ABSTRACTS OF JAPAN vol. 013, no. 394 (C-631), 31 Août 1989 & JP 01 139591 A (TAKEDA CHEM IND LTD), 1 Juin 1989,
- THE JOURNAL OF NUCLEAR MEDICINE, vol. 27, no. 2, Février 1986, pages 235-238, XP000675995 K. HAMACHER ET AL: "Efficient stereospecific synthesis of no-carrier-added 2-[18F]-fluoro-2-deoxy-D-glucose using aminopolyether supported nucleophilic substitution." cité dans la demande
- INT. J. APPL. RADIAT. ISOT., vol. 34, no. 6, 1983, pages 893-896, XP000676201 M. DIKSIC & D. JOLLY: "New high-yield synthesis of 18F-labelled 2-deoxy-2-fluoro-D-glucose."
- JOURNAL OF LABELLED COMPOUNDS AND RADIOPHARMACEUTICALS, vol. 25, no. 7, 1988, pages 721-729, XP000675934 T. HARADAHIRA ET AL: "A new synthesis of 2-deoxy-2-[18F]fluoro-D-galactose using [18F]fluoride ion."

## Description

### Objet de l'invention

La présente invention concerne un nouveau procédé de synthèse de composés marqués tels que le 2-[¹⁸F]fluoro-2-désoxy-D-glucose, plus communément appelé fluoro-désoxy glucose ou FDG.

L'invention concerne également un dispositif de synthèse desdits composés marqué qui repose sur ce procédé, rendant possible une opération automatisée incorporant éventuellement un kit d'équipement à usage unique.

### Arrière-plan technologique

Le FDG est un marqueur dont l'usage est en croissance en imagerie médicale nucléaire. Cette molécule marquée avec le radionucléide ¹⁸F se comporte de façon analogue au glucose lors de la première étape de sa métabolisation dans le corps humain et permet de visualiser et de quantifier ce mécanisme fondamental. Elle est indiquée pour le diagnostic de nombreux dysfonctionnements.

La méthode de synthèse du FDG la plus répandue est la méthode dite de Hamacher, qui est décrite dans Hamacher K., Coenen H. et Stöcklin G., Efficient Stereospecific Synthesis of No-carrier-added 2-[¹⁸F]fluoro-2-désoxy-D-glucose Using Aminopolyether Supported Nucleophilic Substitution, Journal of Nuclear Medicine 27, 235 (1986). Plusieurs variantes de cette méthode ont été mises au point et.sont actuellement exploitées dans divers laboratoires de Tomographie par Emission de Positrons (TEP).

La synthèse repose essentiellement sur les étapes opératoires suivantes-:

### Préparation de l'agent de fluoration

Dans une première étape, le ¹⁸F- est activé au moyens d'agents "activateurs" comme le Kryptofix ® (ou K2.2.2) de manière à le rendre plus réactif. Dans certaines publications on les appelle "agents de transfert de phase". Le radionucléide est produit au préalable, généralement par irradiation d'eau enrichie en oxygène-18 avec un faisceau de protons issu d'un accélérateur de particules, sous forme F⁻ (par exemple H¹⁸F en solution aqueuse).

### Marquage du précurseur

Cet agent de fluoration, rendu totalement anhydre par ajouts d'acétonitrile (CH₃CN) et évaporations à sec, est mis en présence d'un substrat de marquage (ou précurseur), généralement le 1,3,4,6-tétra-O-acétyl-2-trifluorométhanesulfonyl-β-D-mannopyranose (plus communément appelé "triflate") solubilisé dans de l'acétonitrile. Une réaction de substitution s'ensuit où le groupement trifluorométhane sulfonate du substrat (c'est-à-dire du "triflate") est remplacé par l'atome ¹⁸F pour former du 2-[¹⁸F]fluoro-1,3,4,6-tétra-O-acétyl-D-glucose (ou tétraacétylfluoroglucose ou TAFg sous la forme abrégée).

### Pré-purification

Les résidus de réactifs, et en particulier, le kryptofix ® K2.2.2 sont éliminés en passant la solution à travers un "Sep-Pak ® C18" sur lequel le TAFg est fixé. Ce Sep-Pak ® est ensuite rincé avec 10 ml d'HCl 0.1 M. L'activité est ensuite désorbée par 2 ml de tétrahydrofuranne (ou THF) vers le réacteur où celui-ci est totalement éliminé par évaporation.

### Déprotection, en l'occurrence hydrolyse

Le précurseur, en l'occurrence le TAFg, est transformé en FDG par l'élimination de ses quatre groupements acétyles : ces groupements acétyles sont éliminés par une hydrolyse acide réalisée à chaud en solution aqueuse (2 ml HCl 1M à environ 130 °C pendant 15 min.).

### Mise en solution injectable

La solution obtenue, contenant le FDG, est rendue injectable par passage à travers une colonne retardatrice d'ions qui en réduit l'acidité, suivie de Sep-Pak ® alumine et C-18, qui retiennent d'autres impuretés et d'un filtre pour la stérilité. Elle est rendue isotonique par adjonction de NaCl en quantité adéquate. Elle est prête pour le contrôle de qualité ou pour d'autres traitements de préparation à l'administration.

Cette technique comporte cependant une série d'inconvénients, dont les principaux sont :
- la durée de cette procédure est de l'ordre d'une cinquantaine de minutes, notamment en raison du nombre d'opérations de chauffe ou d'évaporation successives, ce qui entraîne une perte d'activité de 30% du simple fait de la demi-vie du ¹⁸F qui est de 110 minutes.
- l'automatisation de cette procédure demande un équipement relativement compliqué et se prête d'autant plus difficilement à la réalisation d'un dispositif à usage unique.

Une étude récente de Mulholland G. Keith (Simple Rapid Hydrolysis of Acetyl Protecting Groups in the FDG Synthesis Using Cation Exchange Resins, Nucl. Med. Biol. Vol. 22, No. 1, pp. 19-23 (1995)) concernant l'hydrolyse a montré que l'acide HCl 1M pouvait être remplacé par une résine cationique présentant des fonctionnalités équivalentes. La résine sulfonique acide sous forme H⁺ (Dowex ® 50) préalablement humidifiée, est placée dans le réacteur d'hydrolyse avant de commencer la synthèse. Le tétraacétylfluoroglucose (TAFg) résultant des étapes précédentes de la synthèse, solubilisé dans de l'éther est introduit dans le réacteur, sur la résine. L'éther est évaporé en 3 à 5 minutes. Le réacteur est chauffé à 100 °C pendant 8 à 10 minutes. Au cours de la dernière minute, on ajoute sur la résine 2 ml d'eau. La solution extraite du réacteur contient le FDG directement disponible à un pH de l'ordre de 4 à 5.5.

Cette méthode qui, à notre connaissance, n'est pas exploitée pour l'utilisation *in vivo,* montre que l'hydrolyse acide peut être réalisée à sec sur support solide. Son principal intérêt tient au fait qu'elle rend inutile la purification au travers d'une colonne retardatrice d'ion. En effet, l'acidité de la solution de FDG obtenue est très faible et se situe dans les limites injectables.

La méthode ne représente cependant aucun gain de temps par rapport à l'hydrolyse "classique" de Hamacher (11-15 min). La suppression d'un réactif, l'HCl 1M, pour l'hydrolyse est compensée par l'adjonction de la résine dans le réacteur d'hydrolyse, résine qui de surcroît doit être conditionnée avant usage. Cette méthode maintient la nécessité de chauffage et d'évaporation des solvants.

Une autre étude récente de F. Füchtner et al. (Basic Hydrolysis of 2-[¹⁸F]fluoro-1,3,4,6-tétra-O-acetyl-D-glucose in the Preparation of 2-[¹⁸F]fluoro-2-deoxy-D-glucose, Appl. Radiat. Isot., Vol. 47, No. 1, pp. 61-66 (1996)) a montré que l'hydrolyse, réalisée habituellement en milieu acide, pouvait être réalisée en milieu basique beaucoup plus rapidement et à température ambiante.

La solution résultant de l'étape de marquage est évaporée à sec, ne laissant que le TAFg (et d'autres résidus non volatils) sur les parois du réacteur de marquage. Une solution aqueuse de NaOH (de préférence 2 ml, 0.3M) est transférée dans le réacteur non chauffé. Après 2 minutes, le TAFg est hydrolysé en FDG avec un rendement de l'ordre de 80%. L'avantage principal de ce procédé est la rapidité de la réaction : 2 minutes au lieu de 8 à 10 minutes pour l'hydrolyse acide.

En pratique cependant, le fait que la réaction puisse être réalisée à température ambiante ne constitue pas un avantage dans un dispositif en réacteur, car avant (ou pendant) hydrolyse, il sera de toute façon nécessaire d'inclure un dispositif de chauffage pour évaporer l'acétonitrile (ou l'éther) provenant de l'étape de marquage et qui accompagne nécessairement le TAFg.

KING-MORRIS M.J. et al. (Stable Isotopically Substituted Carbohydrates: An Improvéd Synthesis of (6-¹³C)Aldohexoses, Carbohydrate Research 175 (1988) 49-58) décrivent un procédé de synthèse d'aldohexoses marqués par l'isotope non-radioactif ¹³C, via réduction de cyanohydrines, comportant les étapes de :
- préparation de l'agent de marquage K¹³CN ;
- marquage d'un précurseur (1,2-O-Isopropylidène-α-D-xylopentodialdo-1,4-furanose) sous forme d'un substrat protégé, la solution de marquage étant diluée avec une solution aqueuse d'acide acétique ;
- réduction préliminaire du composé protégé marqué par H₂ et Pd-BaSO₄, les aldéhydes résultant de cette réaction étant ensuite réduits avec NaBH₄ ;
- déprotection en phase liquide des groupements alcools par hydrolyse acide des composés obtenus ;
- séparation sur résine échangeuse d'ions Dowex 50 X-8 sous forme calcium et
- mise en solution aqueuse et purification des composés marqués.

Le document EP-A-0 798 307 propose alternativement un procédé de synthèse de composés marqués tels que le FDG où l'étape de déprotection fait intervenir une résine échangeuse de cations H⁺ comme réactif pour l'étape de déprotection. Les étapes de synthèse des composés marqués sont les suivantes :
- préparation de l'agent de marquage ;
- marquage du précurseur protégé 1,3,4,6-tétra-O-acétyl-2-trifluorométhane-sulfonyl-β-D-mannopyranose ;
- mise en contact du précurseur protégé marqué avec la résine échangeuse de cations et évaporation des solvants ;
- déprotection par hydrolyse acide du FDG protégé au moyen des ions H⁺ de la résine échangeuse de cations et
- dissolution du FDG marqué dans de l'eau et purification de cette solution de FDG.

La résine échangeuse de cations mentionnée ci-dessus ne présente aucune affinité pour le produit intermédiaire de réaction marqué ou pour la molécule déprotégée. Elle ne sert donc pas à fixer le précurseur protégé marqué pendant la pré-purification et l'hydrolyse, mais comme réactif d'hydrolyse en fournissant les ions H⁺.

Le document EP-A-0 798 307 appartient à l'état de la technique visé à l'Art.54(3) CBE et est donc pris en considération uniquement pour l'appréciation de la nouveauté, et non de l'activité inventive en Allemagne et Grande-Bretagne.

### Buts de l'invention

La présente invention vise à fournir un procédé perfectionné permettant de résoudre les difficultés et inconvénients précités, et vise en particulier à :
- réduire la durée et la complexité de la synthèse, et
- simplifier l'appareillage.

Le procédé selon la présente invention a tout particulièrement pour objectif de simplifier la procédure pour rendre plus facile son automatisation et de réduire la durée de la synthèse pour améliorer son rendement, tout en maintenant, voire en améliorant, le rendement chimique du processus de synthèse.

Des avantages spécifiques au procédé et au dispositif de l'invention sont indiqués ci-après en référence à la description de formes d'exécution possibles de l'invention.

### Eléments caractéristiques de l'invention

Le procédé selon l'invention concerne les méthodes de synthèse de composés marqués avec un quelconque élément isotopique et utilisable notamment dans le domaine médical (RMM, thérapie, imagerie médicale, ...), basées sur le marquage d'un substrat organique dont les groupes fonctionnels déterminés sont préalablement protégés au moyen de groupements protecteurs qui, après marquage, peuvent être éliminés facilement par hydrolyse.

Le procédé est caractérisé en ce que l'élimination de ces groupements protecteurs dans l'étape de déprotection est réalisée directement sur un support solide, compris dans une colonne ou cartouche, présentant une forte affinité pour la molécule protégée et une affinité faible pour la molécule déprotégée.

On entend par "groupes fonctionnels", des fonctions telles que alcool, thiol, phénol, thiophénols, amines, cétones, aldéhydes, acides carboxyliques, etc.

On entend par "groupements protecteurs", des groupements (suivant la fonction à protéger) tels que acétyle, éthers, esters, thioesters, thioéthers, imines, enamines, amides, carbamates, N-Alkyles, N-Aryles, N-hétéro dérivés, cétales, acétales, etc.

On entend par -"colonne" ou "cartouche" toute forme de conditionnement à phase stationnaire utilisable en chromatographie et incluant éventuellement des conteneurs en plastique ou en verre, des colonnes, etc.

Ces produits sont vendus dans le commerce et sont en particulier utilisés en SPE (Solid Phase Extraction) ou chromatographie en phase stationnaire solide.

Selon une forme d'exécution préférée de l'invention, telle qu'appliquée à la synthèse du FDG d'après la méthode de Hamacher, la déprotection est réalisée directement sur la colonne ou cartouche de support solide ayant servi à la pré-purification ou la reformulation du précurseur marqué (élimination des résidus de réactifs et élimination de l'acétonitrile).

La cartouche utilisée peut être par exemple de type C18, C8, tC18, NH2, Diol, polystyrène divinylbenzène (SDB) ou autres phases polymériques telles que disponibles par exemple sous les marques suivantes :
Cartouches Maxi-clean ™ de Alltech :
   - cartouche C18 300 mg (Alltech numéro 20922)
   - cartouche C8 300 mg (Alltech numéro 20946)
   - cartouche NH2 300 mg (Alltech numéro 210040)
   Ces cartouches existent également en versions 600 mg et 900 mg.
Cartouches Waters de 50 mg à 10 g, en particulier :
   - cartouches C18 de type Sep-Pak short body (Waters numéro WAT 020 515)
   - cartouches tC18 (trifonctionnelle) de type Sep-Pak short body de 400 mg (Waters numéro WAT 036 810)
   - cartouche d'extraction Waters OASIS HLB®
Cartouches Varian :
   - Microbond Elut C18 (référence 1214-4002)
   - Microbond Elut C8 (référence 1214-4405)
   - Microbond Elut PS-SDB (référence 1214-4011)
Cartouches Macherey-Nagel :
   - Chromabond C18 500 mg (référence 730 003)
   - Chromabond Phenyl 500 mg (référence 730 084)

Les cartouches et colonnes utilisées contiennent entre 50 mg et 10 g de support solide. Les quantités préférées sont de 200 à 800 mg. D'autres quantités sont également possibles.

Selon l'invention, le support solide est de type phase normale, inverse ou de polarité intermédiaire.

Selon l'invention, le support solide peut être sous forme de grains, de membranes, de feuilles et/ou de capillaires.

Par exemple dans le cas d'une déacétylation comme celle du TAFg , le support solide est de préférence de faible polarité.

En outre, pendant l'étape de déprotection du précurseur, le support solide est avantageusement imprégné par un agent permettant ou accélérant cette déprotection. Cet agent de déprotection peut être une solution aqueuse, de préférence basique ou acide.

Selon une autre variante d'exécution, l'agent de déprotection est le support solide lui-même.

Ladite solution aqueuse basique est de préférence une solution de NaOH et ladite solution aqueuse acide est de préférence une solution de HCl. Selon cette forme d'exécution, le support solide contenu dans la colonne ou la cartouche lors de l'étape de déprotection reste imprégné par la solution acide ou basique pendant 1 à 5 minutes. De plus, on utilise de préférence des concentrations en NaOH ou en HCl dans les solutions basique ou acide comprises entre 0,25 et 2M, de préférence une solution aqueuse acide dont la concentration est comprise entre 0,2 et 2M.

Selon une forme d'exécution préférée de l'invention, après l'étape de déprotection, la cartouche ou la colonne est éluée par un éluant choisi parmi le groupe constitué par de l'eau, une solution aqueuse ou une solution physiologique et ledit éluant entraîne le composé marqué dans le solution et celle-ci est recueillie et éventuellement purifiée, filtrée ou stérilisée.

De préférence, l'étape de marquage est diluée avec une solution acide, de préférence de l'HCl à une concentration comprise entre 0,1 et 1M.

Le précurseur marqué se fixe plus efficacement sur le support solide en milieu aqueux ou en milieu aqueux contenant une proportion faible de solvant organique. Dès lors, la solution de TAFg dans l'acétonitrile (résultant de l'étape de marquage du procédé de Hamacher) est avantageusement diluée avec de l'eau dans une proportion de 10 parties d'eau pour 1 partie de solvant. D'autres dilutions sont également possibles; des dilutions à partir de 5:1 permettent une fixation satisfaisante du TAFg. Il est également apparu qu'il peut être avantageux de diluer la solution de marquage avec une solution acide, par exemple de l'HCl 0,1M, pour améliorer et simplifier l'efficacité de la pré-purification dans le cas où cela est utile, ou toute autre solution aqueuse.

Le mélange est transféré à travers la colonne ou la cartouche de support et ensuite rejeté. Le TAFg, ainsi que certains de ses dérivés, notamment les variétés de TAFg partiellement déacétylés, sont retenus sur le support. Bien entendu, le volume de la colonne ou la cartouche et la quantité de support solide qu'elle contient doivent être suffisants pour retenir l'essentiel du TAFg et de ses dérivés.

La colonne ou la cartouche est ensuite rincée avec une solution aqueuse, soit neutre, soit légèrement acide. Ces rinçages ont pour objectif d'entraîner vers le rejet les traces d'acétonitrile ainsi que de certains résidus de l'étape de marquage présents dans le premier transfert à travers la colonne ou la cartouche.

On notera que le rinçage avec l'HCl 0.1M est facultatif : il dépend de la nature des réactifs utilisés dans l'étape de marquage ou de la composition du diluant ajouté dans la solution marquée avant son transfert à travers la colonne ou la cartouche.

Dans son application à la synthèse du FDG et pour autant qu'un support de type C18 soit utilisé, la procédure décrite jusqu'à ce point est globalement conforme au procédé Hamacher.

Une solution de NaOH est transférée rapidement (quelques secondes) sur la colonne. Son volume est au moins celui que peut contenir la colonne. Le volume transféré sur la colonne peut dépasser le volume de celle-ci, auquel cas l'excès est envoyé au rejet. L'expérience a montré que cet excès n'entraîne qu'une quantité négligeable des composés utiles vers le rejet.

Le support solide reste généralement sous NaOH pendant 1 à 5 minutes. C'est pendant cette période qu'à lieu la déprotection (hydrolyse). La déacétylation complète du TAFg fixé sur le support solide donne lieu au FDG, qui n'a aucune affinité pour le support solide, et se retrouve donc dans la solution qui baigne le support solide. Des concentrations en NaOH comprises entre 0.2M et 2M ont été expérimentées avec succès (concentration optimale variable suivant le support utilisé). D'autres concentrations sont également envisageables, y compris une concentration nulle sur certains supports si l'hydrolyse peut être spontanée et rapide, sans ajout de NaOH, ce qui simplifierait considérablement la purification ultérieure.

La molarité en NaOH de la solution et le volume de la colonne définissent la quantité totale de soude dans la colonne ou la cartouche. L'optimisation à ce niveau consiste à réduire cette quantité totale en vue de faciliter plus tard la neutralisation (ou l'élimination) de la soude.

La durée pendant laquelle la colonne ou la cartouche reste remplie de la solution est celle nécessaire pour obtenir une déprotection quasi-complète du TAFg de départ. Elle dépend notamment de la molarité.

Comme alternative à l'utilisation d'une solution de NaOH, une solution d'HCl peut être transférée sur la colonne, et la déprotection peut être également réalisée sur le support solide de manière semblable. La réalisation complète de l'hydrolyse peut demander que la colonne ou la cartouche soit chauffée.

La colonne ou la cartouche est ensuite éluée par un éluant choisi parmi le groupe constitué par de l'eau, une solution aqueuse ou une solution physiologique. Le FDG est entraîné dans la solution. Celle-ci est transférée vers le flacon final au travers d'éléments tels que : colonne retardatrice d'ions, Sep-Pak ® Al₂O₃, C18, filtre destinés à la purifier et à la stériliser. L'élution peut être effectuée avec une solution de HCl en vue de neutraliser le NaOH (ou l'inverse en cas d'hydrolyse acide). Dans ce cas, l'utilisation d'une colonne retardatrice d'ion est évitée. La mise à pH et isotonicité injectables de la solution finale est assurée par l'ajout d'un tampon. Ce tampon peut être une solution de citrate ou de phosphate de sodium, de tris ou tout autre tampon injectable.

La nature et Les dimensions des éléments de purification dépendront notamment de la quantité de soude ou d'acide mis en oeuvre pour réaliser la déprotection (hydrolyse) .

Les avantages principaux apportés par la déprotection dans une colonne ou une cartouche de support solide sont les suivants :
- à partir de l'étape de marquage, il n'y a plus aucune évaporation nécessaire;
- aucun solvant intermédiaire n'est utilisé (éthanol dans la synthèse de Hamacher, ou éther dans celle de Mulholland et des fabricants d'équipement CTI ou IBA) pour effectuer la pré-purification;
- la déprotection basique dans la colonne ou la cartouche est rapide et ne demande pas de chauffage;
- on évite l'utilisation d'un réacteur pour la déprotection;
- la quantité totale de base (ou d'acide) nécessaire à la réalisation de la déprotection est réduite au volume de la colonne ou la cartouche. Son élimination est de ce fait simplifiée. Alternativement, sa neutralisation est rendue possible par l'ajout de faibles volumes d'acide (ou de base) et de tampon;
- l'utilisation d'une colonne retardatrice d'ions est évitée.

La déprotection, par exemple par hydrolyse, dans un élément de support solide, et en particulier dans celui utilisé pour la pré-purification, permet donc de raccourcir la durée de la synthèse, de réduire le nombre de réactifs, et de réduire le nombre de composants d'un dispositif de production des composés marqués. Cette simplification rend la technique plus aisément transposable à l'utilisation d'un dispositif de synthèse automatisé incorporant un kit à usage unique.

Un dernier aspect de la présente invention concerne le dispositif de synthèse du 2-[¹⁸F]fluoro-2-désoxy-D-glucose (FDG) par le procédé selon l'invention, dans lequel on utilise un support solide dans l'étape de déprotection, de préférence sous forme d'un kit d'équipement à usage unique.

Avantageusement, ce dispositif est automatisé.

### Description d'une forme d'exécution préférée de l'invention

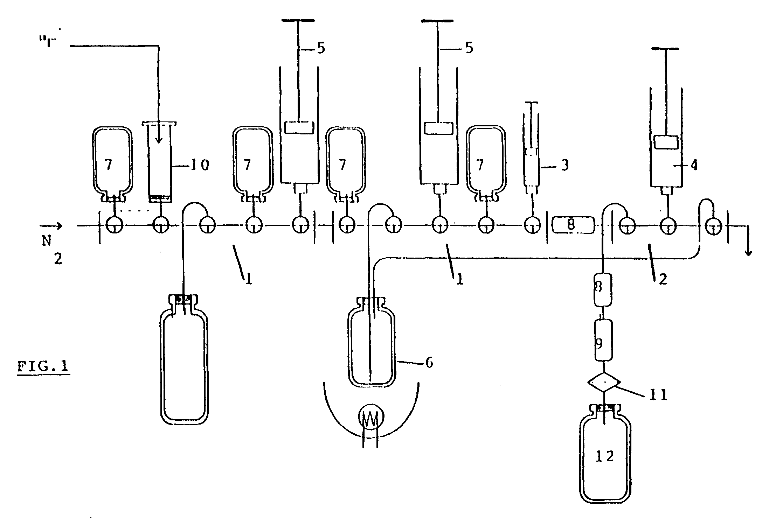

L'invention sera décrite plus en détails en référence à un exemple spécifique d'exécution illustré schématiquement dans la figure 1 annexée.

### Exemple : synthèse de 2-[¹⁸F]fluoro-2-désoxy-D-glucose en utilisant des rampes de vannes et des seringues stériles jetables

La synthèse de 2-[¹⁸F]fluoro-2-désoxy-D-glucose (ou FDG) réalisée dans le module dont le schéma est repris en annexe (figure 1) comprend les étapes suivantes:
- récupération de l'eau enrichie en oxygène 18,
- séchage de l'agent de fluoration [K/222]⁺¹⁸ F⁻,
- marquage du triflate,
- pré-purification,
- déprotection (hydrolyse) en milieu basique sur support solide, et
- formulation de la solution injectable.

Cette synthèse est réalisée dans un kit disposable unique (figure 1) comprenant les composants à usage unique suivants :

| **Composants** | **Marque ou fournisseur** | **Référence** | **Qté** |
|---|---|---|---|
| Rampe 5 vannes (1) | PVB | 888-105 | 2 |
| Rampe 3 vannes (2) | PVB | 888-103 | 1 |
| Seringue 2 ml (3) | Terumo | BS-02S | 1 |
| Seringue 20 ml (4) | Plastipak | NO. 300134 | 1 |
| Seringue 60 ml (5) | Plastipak | NO. 300856 | 2 |
| Réacteur (6) | Alltech | 66124 | 1 |
| Flacons de réactifs (7) | Alltech | 6655 | 4 |
| Capsule de fixation | Alltech | 66440 | 1 |
| Septum | Alltech | 95313 | 1 |
| Cartouches tC18 (8) | Waters | 36810 | 2 |
| Cartouche alumine | Waters | 20510 | 1 |
| Cartouche SAX (M. PORE) (10) | Varian/3M | | 1 |
| Filtre 0,22 µm (11) | Millipore | SVGS0250S | 1 |
| Fiole stérile sous vide (12) | Mallinckrodt | DRN4370 | 1 |
| Prolongateur | Vigon | 110901 | 2 |
| Aiguille | B. Braun | 466 579/1 | 1 |

Les étapes opératoires suivantes permettent l'obtention du FDG via l'utilisation du système décrit ci-dessus. La réaction de synthèse se déroule comme suit :
1. Récupération de l'eau enrichie en oxygène-18 au moyen d'une résine anionique
2. Récupération par élution de la résine anionique, de l'activité sous forme de [K/222]¹⁸⁺ F⁻ en solution dans un mélange CH₃CN / H₂O
3. Evaporation du solvant par chauffage au rayonnement infrarouge (105 °C) sous flux d'azote (2 min 30 sec)
4. Ajout de 1 ml de CH₃CN, évaporation (2 min 30 sec)
5. Ajout de 1 ml de CH₃CN, évaporation à sec (détermination de la fin de l'évaporation grâce à une sonde de température)
6. Refroidissement du réacteur à 70 °C
7. Ajout d'une solution de précurseur de marquage (15 mg) dans du CH₃CN (1,7 ml)
8. Chauffage à 95 °C pendant 3 min (c'est l'étape de marquage)
9. Dilution de la solution résultante dans 25 ml d'eau
10. Passage de la solution diluée à travers une cartouche C18 (préalablement conditionnée par 5 ml d'éthanol et 10 ml d'eau) et envoi au rejet
11. Rinçage de la cartouche par 10 ml d'HCl 0,1 N et 10 ml d'eau qui sont envoyés au rejet
12. Séchage de la cartouche sous flux d'azote
13. Ajout de 0.7 ml de NaOH 1.5M sur la cartouche C18
14. Déprotection (hydrolyse) 1.5 min à température ambiante
15. Elution du FDG par 5 ml d'eau dans une seringue contenant 0.8 ml d'HCl 1.5M et 5 ml de tampon citrate
16. Passage de la solution résultante à travers une cartouche C18, une cartouche alumine neutre et un filtre 0,22 µm; la solution est récupérée dans une fiole stérile

## Revendications

1. Procédé de synthèse du composé marqué 2-[¹⁸F]fluoro-2-désoxy-D-glucose ([¹⁸F]FDG), comportant les étapes de :
- préparation de l'agent de marquage,
- marquage au moyen du radionucléide ¹⁸F d'un précurseur se trouvant sous la forme d'un substrat protégé,
- purification préliminaire du précurseur marqué,
- déprotection du précurseur marqué et
- mise en solution finale,
**caractérisé en ce que** l'étape de déprotection, c'est-à-dire la transformation du précurseur marqué en composé marqué, est réalisée directement sur support solide ou sur phase stationnaire solide, se présentant de préférence sous la forme de grains, de membranes, de feuilles et/ou de capillaires, contenus dans une colonne ou une cartouche, de type C18, C8, tC18, NH2, Diol, polystyrène divinylbenzène (SDB), Phényl ou Waters OASIS HLB®.

2. Procédé selon la revendication 1, **caractérisé en ce que** l'on utilise une colonne ou une cartouche de type. commercial telle qu'utilisée dans la technique dite SPE (Solid Phase Extraction).

3. Procédé selon la revendication 1 ou 2, **caractérisé en ce que** l'étape de déprotection est réalisée directement dans la colonne ou la cartouche de support solide ayant servi à la purification préliminaire.

4. Procédé selon l'une quelconque des revendications précédentes, **caractérisé en ce que**, pendant l'étape de déprotection du précurseur, le support solide est imprégné par un agent permettant ou accélérant cette déprotection.

5. Procédé selon l'une quelconque des revendications 1 à 3, **caractérisé en ce que** l'agent de déprotection est le support solide lui-même.

6. Procédé selon l'une quelconque des revendications 1 à 4, **caractérisé en ce que** la déprotection est réalisée par saponification, de préférence à l'aide d'une solution aqueuse de NaOH ayant avantageusement une concentration comprise entre 0,2 et 2M.

7. Procédé selon l'une quelconque des revendications 1 à 4, **caractérisé en ce que** la déprotection est réalisée par hydrolyse acide, de préférence à l'aide d'une solution aqueuse de HCl ayant avantageusement une concentration comprise entre 0,2 et 2M.

8. Procédé selon l'une quelconque des revendications 1 à 4, 6 et/ou 7, **caractérisé en ce que** le support solide contenu dans la colonne ou la cartouche lors de l'étape de déprotection reste en milieu acide ou basique pendant 1 à 5 minutes.

9. Procédé selon l'une quelconque des revendications précédentes, **caractérisé en ce qu'**après l'étape de déprotection, la cartouche ou la colonne est éluée par un éluant choisi parmi le groupe constitué par de l'eau, une solution aqueuse ou une solution physiologique, **en ce que** ledit éluant entraîne le composé marqué dans la solution et que celle-ci est recueillie et éventuellement purifiée, filtrée ou stérilisée.

10. Procédé selon la revendication 9, **caractérisé en ce que** la solution de l'étape de marquage est diluée avec une solution acide, de préférence de l'HCl, et à une concentration comprise entre 0,1 et 1M.

11. Procédé selon l'une quelconque des revendications précédentes, **caractérisé en ce que** le précurseur protégé est le 1,3,4,6-tétra-O-acétyl-2-trifluorométhanesulfonyl-β-D-mannopyranose (triflate).

12. Procédé selon l'une quelconque des revendications précédentes, **caractérisé en ce que** le support solide est de faible polarité.

13. Dispositif de synthèse du 2-[¹⁸F]fluoro-2-désoxy-D-glucose ([¹⁸F]FDG) par le procédé selon l'une quelconque des revendications précédentes, **caractérisé en ce qu'**il comporte un support solide pour réaliser l'étape de déprotection.

14. Dispositif selon la revendication 13, **caractérisé en ce que** le dispositif incorpore un élément à usage unique incluant ledit support solide.

15. Dispositif selon la revendication 13 ou 14, **caractérisé en ce qu'**il est automatisé.

## Patentansprüche

1. Verfahren zur Herstellung der markierten Verbindung 2-[¹⁸F] Fluor-2-desoxy-D-glucose ([¹⁸F] FDG) , das folgende Schritte umfasst:
- Vorbereitung einer Markierungssubstanz,
- Markierung einer Vorläufersubstanz (Präkursor) in geschütztem Zustand durch das Radionuklid ¹⁸F,
- vorbereitende Reinigung des markierten Präkursors,
- Entschützung des markierten Präkursors und
- abschließende Lösung,
**dadurch gekennzeichnet, dass** das Entschützen, also die Verwandlung des markierten Präkursors in eine markierte Verbindung, direkt auf einem festen Träger oder einer festen stationären Phase vorgenommen wird, die vorzugsweise die Form von Körnern, Membranen, Blättern und/oder Kapillaren aufweisen und in einer Säule oder Kartusche vom Typ C18, C8, tC18, NH2, Diol, Polystyrol-Divinylbenzol (SDB), Phenyl oder Waters OASIS HLB® enthalten sind.

2. Verfahren nach Anspruch 1, **dadurch gekennzeichnet, dass** man eine handelsübliche Säule oder Kartusche, wie sie bei der SPE-Methode (Solid Phase Extraction) verwendet wird, benutzt.

3. Verfahren nach Anspruch 1 oder 2, **dadurch gekennzeichnet, dass** das Entschützen direkt in der Säule oder Kartusche des festen Trägers vorgenommen wird, in der auch die vorbereitende Reinigung stattgefunden hat.

4. Verfahren nach einem der obenstehenden Ansprüche, **dadurch gekennzeichnet, dass** der feste Träger während des Entschützens des Präkursors mit einer Substanz getränkt ist, die das Entschützen ermöglicht oder beschleunigt.

5. Verfahren nach einem der Ansprüche 1 bis 3, **dadurch gekennzeichnet, dass** der feste Träger selbst die entschützende Substanz ist.

6. Verfahren nach einem der Ansprüche 1 bis 4, **dadurch gekennzeichnet, dass** das Entschützen durch Verseifung vorgenommen wird, vorzugsweise mit einer wässrigen NaOH-Lösung, idealerweise in einer Konzentration von 0,2 bis 2M.

7. Verfahren nach einem der Ansprüche 1 bis 4, **dadurch gekennzeichnet, dass** das Entschützen durch saure Hydrolyse erfolgt, vorzugsweise mit einer wässrigen HCl-Lösung, idealerweise in einer Konzentration von 0,2 bis 2M.

8. Verfahren nach einem der Ansprüche 1 bis 4, 6 und/oder 7, **dadurch gekennzeichnet, dass** der während des Entschützens in der Säule oder Kartusche enthaltene feste Träger 1 bis 5 Minuten lang in einem sauren oder basischen Milieu bleibt.

9. Verfahren nach einem der obenstehenden Ansprüche, **dadurch gekennzeichnet, dass** die Säule bzw. Kartusche nach dem Entschützen mit einem Eluierungsmittel, das aus der Gruppe Wasser, wässrige Lösung oder physiologische Lösung gewählt wird, eluiert wird und das besagte Eluierungsmittel die markierte Verbindung in die Lösung zieht und diese Lösung aufgefangen und eventuell gereinigt, gefiltert oder sterilisiert wird.

10. Verfahren nach Anspruch 9, **dadurch gekennzeichnet, dass** die Lösung der Markierung mit einer sauren Lösung verdünnt wird, vorzugsweise mit HC1, idealerweise in einer Konzentration von 0,1 bis 1M.

11. Verfahren nach einem der obenstehenden Ansprüche, **dadurch gekennzeichnet, dass** der geschützte Präkursor 1,3,4,6-Tetra-O-acetyl-2-Otrifluormethansulphonyl-beta-D-mannopyranose (Triflat) ist.

12. Verfahren nach einem der obenstehenden Ansprüche, **dadurch gekennzeichnet, dass** der feste Träger eine schwache Polarität hat.

13. Vorrichtung zur Herstellung von 2-[¹⁸F] Fluor-2-desoxy-D-glucose ([¹⁸F] FDG) gemäß einem Verfahren nach einem der obenstehenden Ansprüche, **dadurch gekennzeichnet, dass** sie über einen festen Träger zur Durchführung des Entschützens verfügt.

14. Vorrichtung nach Anspruch 13, **dadurch gekennzeichnet, dass** die Vorrichtung über ein Einwegelement verfügt, das den besagten festen Träger enthält.

15. Vorrichtung nach Anspruch 13 oder 14, **dadurch gekennzeichnet, dass** sie automatisiert ist.

## Claims

1. Method for synthesising the labelled compound 2-[¹⁸F]fluoro-2-deoxy-D-glucose ([¹⁸F] FDG), comprising the stages of:
- preparation of the labelling agent,
- labelling by means of the radionuclide ¹⁸F of a precursor present in the form of a protected substrate,
- preliminary purification of the labelled precursor,
- deprotection of the labelled cursor, and
- final solution treatment,
**characterised in that** the stage of deprotection, i.e. the transformation of the labelled precursor into a labelled compound, occurs directly on a solid support or on a solid stationary phase, preferably being in the form of grains, membranes, films and/or capillaries, contained in a column or a cartridge, of type C18, C8, tC18, NH2, diol, polystyrene divinylbenzene (SDB), phenyl or Waters OASIS HLB®.

2. Method as in Claim 1, **characterised in that** a column or cartridge of a commercial type such as used in the technique known as SPE (Solid Phase Extraction) is used.

3. Method as in Claim 1 or Claim 2, **characterised in that** the stage of deprotection occurs directly in the column or the cartridge of the solid support used for the preliminary purification.

4. Method as in any of the preceding claims, **characterised in that**, during the deprotection phase of the precursor, the solid support is impregnated with an agent that allows or accelerates this deprotection.

5. Method as in any of Claims 1 to 3, **characterised in that** the deprotection agent is the solid support itself.

6. Method as in any of Claims 1 to 4, **characterised in that** the deprotection is achieved by the saponification, preferably by means of an aqueous solution of NaOH having as an advantage a concentration of between 0.2 and 2M.

7. Method as in any of Claims 1 to 4, **characterised in that** the deprotection is achieved by acid hydrolysis, preferably by means of an aqueous solution of HCl having as an advantage a concentration of between 0.2 and 2M.

8. Method as in any of Claims 1 to 4, 6 and/or 7, **characterised in that** the solid support contained in the column or the cartridge during the deprotection stage remains in an acid or alkaline medium for 1 to 5 minutes.

9. Method as in any of the preceding claims, **characterised in that**, after the deprotection stage, the cartridge or the column is eluted by an eluent chosen from the group comprising water, an aqueous solution or a physiological solution, **in that** said eluent carries the labelled compound into the solution and **in that** the latter is collected and possibly purified, filtered or sterilised.

10. Method as in Claim 9, **characterised in that** the solution of the labelling stage is diluted with an acid solution, preferably HCl, at a concentration between 0.1 and 1M.

11. Method as in any of the preceding claims, **characterised in that that** the protected precursor is 1,3,4,6-tetra-O-acetyl-2-trifluoromethanesulfonyl-β-D-mannopyranose (triflate).

12. Method as in any of the preceding claims, **characterised in that** the solid support is of weak polarity.

13. Device for the synthesis of 2-[¹⁸F]fluoro-2-deoxy-D-glucose ([¹⁸F]FDG) by the method as in any of the preceding claims, **characterised in that** it comprises a solid support for carrying out the deprotection stage.

14. Device as in Claim 13, **characterised in that** the device includes a disposable element including said solid support.

15. Device as in Claim 13 or Claim 14, **characterised in that** it is automated.
